# EUROPÄISCHE PATENTSCHRIFT

(11) **EP 3 789 082 B1**
(45) Veröffentlichungstag und Bekanntmachung des Hinweises auf die Patenterteilung: **26.07.2023**
(21) Anmeldenummer: 19195857.8
(22) Anmeldetag: 06.09.2019
(51) Int. Cl.: A61N 1/37, A61N 1/368, A61B 5/00, A61B 5/347, A61B 5/349

(54) **IMPLANTIERBARE ANORDNUNG ZUR DETEKTION ELEKTRISCHER SIGNALE EINES MENSCHLICHEN ODER TIERISCHEN HERZENS**
IMPLANTABLE ASSEMBLY FOR DETECTING ELECTRICAL SIGNALS OF A HUMAN OR ANIMAL HEART
DISPOSITIF IMPLANTABLE DESTINÉ À LA DÉTECTION DE SIGNAUX ÉLECTRIQUES D'UN COEUR HUMAIN OU ANIMAL

(43) Veröffentlichungstag der Anmeldung: 10.03.2021
(73) Patentinhaber: BIOTRONIK SE & Co. KG, 12359 Berlin (DE)
(72) Erfinder: Becker, Frank, 10409 Berlin (DE)
(74) Vertreter: Biotronik Corporate Services SE

(56) Entgegenhaltungen:
- EP-A1- 2 060 299
- US-A1- 2005 137 632
- US-A1- 2009 112 276
- US-A1- 2010 262 204
- US-A1- 2018 110 980
- US-A1- 2019 046 802

## Beschreibung

Die vorliegende Erfindung betrifft eine implantierbare Anordnung zur Detektion elektrischer Signale eines menschlichen oder tierischen Herzens gemäß dem Oberbegriff des Anspruchs 1, ein Verfahren zur Steuerung des Betriebs einer derartigen Anordnung gemäß dem Oberbegriff des Anspruchs 12 und ein Computerprogrammprodukt gemäß dem Oberbegriff des Anspruchs 13.

Die atrioventrikuläre Überleitungszeit und die interventrikuläre Überleitungszeit sind zwei wichtige Kenngrößen, aus denen sich auf den Zustand eines menschlichen oder tierischen Herzens schließen lässt und die beim Betrieb eines Herzschrittmachers berücksichtigt werden müssen, damit die vom Herzschrittmacher ausgesendeten Impulse in einer wirksamen Stimulation des behandelten Herzens resultieren.

Die atrioventrikuläre Überleitungszeit ist die Zeit, die zwischen einer Stimulation oder intrinsischen Aktivität des Atriums und einer unmittelbar nachfolgenden ventrikulären Aktivität vergeht. Die interventrikuläre Überleitungszeit gibt den zeitlichen Versatz zwischen einer Kontraktion des rechten Ventrikels und der unmittelbar darauffolgenden Kontraktion des linken Ventrikels an. Im gesunden Herzen kontrahieren der rechte Ventrikel und der linke Ventrikel zeitgleich. Bei Patienten mit kardiologischen Erkrankungen kann es jedoch zu einem zeitlichen Versatz kommen. Dadurch lässt die Pumpleistung des Herzens nach, was sich in einer Herzinsuffizienz und in einer Vergrößerung des Herzens bemerkbar macht. Um in einem solchen Fall eine zeitlich besser synchronisierte Kontraktion beider Ventrikel zu erreichen, ist es erforderlich, den zeitlichen Versatz zwischen der Kontraktion des linken Ventrikels und der Kontraktion des rechten Ventrikels sehr genau zu bestimmen. Dies erfolgt typischerweise nach Implantation eines Schrittmachers. Problematisch hieran ist, dass sich die interventrikuläre Überleitungszeit insbesondere in der ersten Zeit nach der Implantation eines Schrittmachers, grundsätzlich aber auch zu einem späteren Zeitpunkt, verändern kann. Dann ist eine Nachjustierung des Schrittmachers erforderlich, um weiterhin eine gute therapeutische Wirksamkeit des Schrittmachers zu erreichen. Bei linksventrikulären Leads mit mehreren Stimulationspolen, wie e.g. quadrupolaren Leads, kann eine Messung der interventrikulären Überleitungszeit zwischen rechtem Ventrikel und jedem der linksventrikulären Stimulationspolen vorgenommen werden. Basierend auf den gemessenen Zeiten wird der optimale Stimulationspol ausgewählt.

Die US 2010/0100148 A1 beschreibt die Möglichkeit, die atrioventrikuläre Überleitungszeit und die interventrikuläre Überleitungszeit im Rahmen eines Reizschwellentests eines Herzschrittmachers zu bestimmen. Problematisch bei dieser Bestimmungsmethode ist indes, dass hierbei eine extrinsische Stimulation des betreffenden Herzens durchgeführt wird, die sich mit der intrinsischen Herzaktivität überlagern kann.

Weitere Vorrichtungen zur Bestimmung einer intrinsischen atrioventrikulären bzw. interventrikulären Überleitungszeit sind bekannt aus US 2019/046802 A1, US 2018/110980 A1, US 2005/137632 A1, US 2009/112276 A1, EP 2 060 299 A1 und US 2010/262204 A1.

Der vorliegenden Erfindung liegt die Aufgabe zugrunde, ein implantierbares Medizinprodukt bereitzustellen, das eine vereinfachte und verlässliche Bestimmung der atrioventrikulären Überleitungszeit und/oder der interventrikulären Überleitungszeit auch nach seiner Implantation ermöglicht.

Diese Aufgabe wird mit einer implantierbaren Anordnung zur Detektion elektrischer Signale eines menschlichen oder tierischen Herzens mit den Merkmalen des Anspruchs 1 gelöst.

Die Erfindung wird durch die nachfolgenden Ansprüche definiert. Nachfolgend beschriebene Aspekte, Ausführungsvarianten, Verfahren und Beispiele die nicht unter den Wortlaut der Ansprüche fallen, sind nicht Teil der Erfindung.

Eine derartige Anordnung weist einen Prozessor, eine Speichereinrichtung, eine erste Detektionseinrichtung, eine zweite Detektionseinrichtung und eine dritte Detektionseinrichtung auf. Die erste Detektionseinrichtung dient zum Detektieren einer atrialen Aktivität, also einer Aktivität in einem Atrium des menschlichen oder tierischen Herzens, insbesondere im rechten Atrium. Die zweite Detektionseinrichtung dient zum Detektieren einer rechtsventrikulären Aktivität, während die dritte Detektionseinrichtung zum Detektieren einer linksventrikulären Aktivität eines menschlichen oder tierischen Herzens dient.

Die Anordnung zeichnet sich erfindungsgemäß dadurch aus, dass die Speichereinrichtung ein computerlesbares Programm aufweist, das den Prozessor dazu veranlasst, die nachfolgend erläuterten Schritte in regelmäßigen Abständen automatisch auszuführen, wenn es auf dem Prozessor ausgeführt wird.

Gemäß einer ersten Variante wird zunächst eine intrinsische rechtsatriale Aktivität mittels der ersten Detektionseinrichtung erfasst. Anschließend wird eine unmittelbar auf die zuvor erfasste rechtsatriale Aktivität folgende intrinsische rechtsventrikuläre Aktivität mittels der zweiten Detektionseinrichtung erfasst. Nachfolgend wird die Zeit, die zwischen der intrinsischen rechtsatrialen Aktivität und der intrinsischen rechtsventrikulären Aktivität vergangen ist, bestimmt. Diese Zeit wird anschließend als atrioventrikuläre Überleitungszeit gespeichert, beispielsweise in der Speichereinrichtung.

Alternativ oder zusätzlich zu den vorstehend erläuterten Schritten kann das Programm den Prozessor dazu veranlassen, die nachfolgend erläuterten Schritte auszuführen.

Gemäß dieser zweiten Variante wird zunächst eine intrinsische rechtsventrikuläre Aktivität mittels der zweiten Detektionseinrichtung erfasst. Anschließend wird eine unmittelbar auf die zuvor erfasste intrinsische rechtsventrikuläre Aktivität folgende intrinsische linksventrikuläre Aktivität mittels der dritten Detektionseinrichtung erfasst. Nachfolgend wird die Zeit, die zwischen der intrinsischen rechtsventrikulären Aktivität und der intrinsischen linksventrikulären Aktivität vergangen ist, bestimmt. Diese Zeit wird dann als interventrikuläre Überleitungszeit gespeichert, beispielsweise in der Speichereinrichtung.

Im Gegensatz zu den aus dem Stand der Technik bekannten Medizingeräten zielt die vorliegend beanspruchte implantierbare Anordnung darauf ab, ausschließlich eine intrinsische Aktivität (Aktion) der einzelnen Kammern eines menschlichen oder tierischen Herzens zu erfassen. Externe Stimuli, die beispielsweise von einer Stimulationseinrichtung eines Herzschrittmachers ausgesendet werden, werden ausdrücklich nicht ausgewertet.

Darüber hinaus ist die vorliegend beanspruchte implantierbare Anordnung dafür ausgelegt, die erläuterten Verfahrensschritte in regelmäßigen Abständen automatisch durchzuführen. Somit können stets verlässliche Daten zur atrioventrikulären Überleitungszeit und/oder zur interventrikulären Überleitungszeit bereitgestellt werden. Diese Daten können dann zur Einstellung der Parameter eines Herzschrittmachers oder einer anderen Vorrichtung zur Stimulation eines menschlichen oder tierischen Herzens verwendet werden. Sie können auch als Ausgangsdaten für nachfolgende diagnostische Untersuchungen eingesetzt werden. Beispielsweise lässt sich bei einer Beobachtung des zeitlichen Verlaufs der atrioventrikulären Überleitungszeit oder der interventrikulären Überleitungszeit eine Veränderung des Zustands des Herzens ermitteln. Ein Arzt kann dann über geeignete Gegenmaßnahmen zu der beobachteten Veränderung des Zustands des Herzens entscheiden. Darüber hinaus kann ein Arzt auf der Grundlage derartiger Daten auch beurteilen, ob eine Vorrichtung zur Stimulation des Herzens korrekt funktioniert und sich in Bezug auf den kardiologischen Zustand des Patienten wie gewünscht verhält.

In einer Variante werden im Rahmen der Bestimmung der interventrikulären Überleitungszeit nicht nur die rechtsventrikuläre Aktivität und die linksventrikuläre Aktivität erfasst. Vielmehr wird in dieser Variante mittels der ersten Detektionseinrichtung auch eine intrinsische rechtsatriale Aktivität erfasst. Dann kann eine besonders verlässliche Aussage darüber getroffen werden, dass die detektierten ventrikulären Aktivitäten tatsächlich durch ein intrinsisches atriales Signal des betrachteten Herzens ausgelöst wurden.

Wenn die intrinsische rechtsatriale Aktivität mittels der ersten Detektionsvorrichtung erfasst wird, kann in einer weiteren Variante zudem zusätzlich die atrioventrikuläre Überleitungszeit bestimmt werden, sodass innerhalb eines Messzyklus sowohl die atrioventrikuläre Überleitungszeit als auch die interventrikuläre Überleitungszeit bestimmt werden. Bei dieser Variante handelt es sich dann folglich um eine Kombination der beiden oben erläuterten Varianten.

Erfindungsgemäß veranlasst das Programm den Prozessor dazu, die oben erläuterten Schritte zur Bestimmung der atrioventrikulären Überleitungszeit und/oder der interventrikulären Überleitungszeit einmal in einem Zeitraum von 3 Stunden bis 48 Stunden, insbesondere von 6 Stunden bis 36 Stunden, insbesondere von 12 Stunden bis 24 Stunden durchzuführen. Beispielsweise kann das Programm den Prozessor dazu veranlassen, die genannten Schritte einmal pro Tag durchzuführen. Dann lassen sich die atrioventrikuläre Überleitungszeit und/oder die interventrikuläre Überleitungszeit besonders einfach in einem definierten Zeitabstand ermitteln und speichern.

In einer Variante ist die implantierbare Anordnung nicht nur zur Detektion elektrischer Signale eines menschlichen oder tierischen Herzens geeignet, sondern auch zur Stimulation desselben menschlichen oder tierischen Herzens vorgesehen und eingerichtet. Dann können die bestimmte atrioventrikuläre Überleitungszeit und/oder die bestimmte interventrikuläre Überleitungszeit besonders einfach zur Anpassung von Parametern (beispielsweise der AV-Verzögerung oder der VV-Verzögerung) desselben Gerätes verwendet werden, das im Bedarfsfall auch eine Stimulation des Herzens durchführt.

In einer Variante handelt es sich bei der implantierbaren Anordnung um einen kardialen Schrittmacher, insbesondere um ein Gerät zur kardialen Resynchronisationstherapie (CRT-Gerät). Denn gerade bei derartigen Herzschrittmachern bzw. CRT-Geräten ist eine genaue Einstellung der bedarfsweise abzugebenden Stimulationsimpulse auf die physiologischen Gegebenheiten im jeweiligen Herz besonders relevant. Daher kann durch die Ermittlung der atrioventrikulären Überleitungszeit und/oder der interventrikulären Überleitungszeit hier eine besonders sinnvolle Parametereinstellung am Herzschrittmacher bzw. CRT-Gerät vorgenommen werden.

In einer Variante veranlasst das Programm den Prozessor dazu, eine atriale Stimulation und eine ventrikuläre Stimulation so lange zu inhibieren, bis die atrioventrikuläre Überleitungszeit und/oder die interventrikuläre Überleitungszeit bestimmt ist. Auf diese Weise wird sichergestellt, dass keine extrinsischen Stimulationssignale erfasst werden können, die von der in dieser Variante als Stimulationseinrichtung ausgestalteten implantierbaren Anordnung grundsätzlich in das betrachtete menschliche oder tierische Herz abgegeben werden können und sich mit den intrinsischen Signalen der Herzaktivität überlagern können. Da die Durchführung der oben erläuterten Schritte zur Bestimmung der atrioventrikulären Überleitungszeit und/oder der interventrikulären Überleitungszeit nur einen sehr geringen Zeitbedarf aufweisen, ist eine Inhibition von Stimulationsimpulsen während der benötigten Messzeit für die Gerätesicherheit bzw. den Gesundheitszustand eines Patienten unproblematisch.

In einer Variante veranlasst das Programm den Prozessor dazu, die oben erläuterten Schritte zur Bestimmung der atrioventrikulären Überleitungszeit und/oder der interventrikulären Überleitungszeit während einer routinemäßig durchgeführten Messung eines Signals der intrinsischen Herzaktivität auszuführen. Erfindungsgemäß werden besagte oben erläuterte Schritte während einer Bestimmung und/oder Anpassung einer Detektionsschwelle der implantierbaren Anordnung ausgeführt.

Typischerweise wird die routinemäßige Messung eines Signals der intrinsischen Herzaktivität bzw. die Bestimmung und/oder Anpassung einer Detektionsschwelle der implantierbaren Anordnung einmal täglich durchgeführt. Während dieser Zeit erfolgt in aller Regel ohnehin eine Unterdrückung der Abgabe von Stimulationsimpulsen. Das von der vorliegend beanspruchten Anordnung durchgeführte Verfahren zur Ermittlung der atrioventrikulären Überleitungszeit und/oder der interventrikulären Überleitungszeit lässt sich in dieser Variante ohne wesentlichen Zeitverlust während der routinemäßigen Messung eines Signals der intrinsischen Herzaktivität bzw. während der Bestimmung und/oder Anpassung einer Detektionsschwelle der implantierbaren Anordnung durchführen. Auf diese Weise lassen sich also dann, wenn die implantierbare Anordnung ohnehin in Bezug auf ihr Ansprechverhalten getestet wird, zusätzlich - jedoch ohne signifikanten zeitlichen Mehraufwand - die atrioventrikuläre Überleitungszeit und/oder die interventrikuläre Überleitungszeit ermitteln. Somit bietet gerade diese Variante eine besonders einfache Möglichkeit zur Realisierung der automatischen regelmäßigen Ermittlung der atrioventrikulären Überleitungszeit und/oder der interventrikulären Überleitungszeit.

Gemäß einer Ausführungsform der erfindungsgemäßen Vorrichtung umfasst die Messung eines Signals der intrinsischen Herzaktivität die Messung und/oder Bestimmung von mindestens einem Signalparameter aus Amplitude, Pulsbreite, Anzahl der Nulldurchgänge, Amplituden- und/oder Phasen-Frequenzspektrum und/oder Energie des Signals sowie die Parameter
- eines Modells des Signals (z.B. ARMA, AutoRegressive-Moving Average) und/oder
- einer Transformation des Signal (z.B. Wavelet)
   und/oder

Amplitude, Pulsbreite, Anzahl der Nulldurchgänge, Amplituden- und/oder Phasen-Frequenzspektrum und/oder Energie einer zeitlichen Ableitung des Signals.

In einer Variante veranlasst das Programm den Prozessor dazu, eine Vielzahl (zwei oder mehr) von zuvor bestimmten atrioventrikulären Überleitungszeiten und/oder interventrikulären Überleitungszeiten zu speichern. Das Speichern kann beispielsweise in der Speichereinrichtung der implantierbaren Anordnung erfolgen. Durch ein derartiges Speichern der zuvor bestimmten Überleitungszeiten lässt sich eine Historie der bereits ermittelten Überleitungszeiten erstellen. Es kann dann beispielsweise ermittelt werden, inwieweit sich eine neu bestimmte Überleitungszeit von in der Vergangenheit ermittelten Überleitungszeiten unterscheidet. Dabei werden typischerweise die beiden Überleitungszeitentypen (also die atrioventrikulären Überleitungszeiten auf der einen Seite und die interventrikulären Überleitungszeiten auf der anderen Seite) getrennt voneinander gespeichert und bei Bedarf mit einer oder mehreren Überleitungszeiten desselben Typs verglichen.

In einer Variante veranlasst das Programm den Prozessor dazu, aus der Vielzahl gespeicherter atrioventrikulärer Überleitungszeiten und/oder interventrikulärer Überleitungszeiten einen zeitlichen Verlauf der jeweiligen Überleitungszeit zu ermitteln. Zu diesem Zweck werden die einzelnen Überleitungszeiten mit einer Zeitinformation in Bezug auf den Zeitpunkt ihrer Bestimmung gespeichert. Dann kann besonders einfach ein zeitlicher Verlauf der entsprechenden Überleitungszeit ermittelt werden. Aus diesem zeitlichen Verlauf kann sich ein Trend einer der beiden Überleitungszeiten oder beider Überleitungszeiten in eine bestimmte Richtung ermitteln, der einen Rückschluss auf eine Veränderung des Zustands des betrachteten Herzens erlaubt. Alternativ oder zusätzlich lassen sich die derart gewonnenen Daten auch dazu verwenden, einen Rückschluss auf die Interaktion zwischen der implantierbaren Anordnung und dem Herzen, dessen Signale erfasst werden, ermitteln.

In einer Variante veranlasst das Programm den Prozessor dazu, die atrioventrikuläre Überleitungszeit und/oder die interventrikuläre Überleitungszeit an ein externes Gerät oder an ein externes Datenzentrum zu übermitteln. Auf diese Weise können die gewonnenen Daten besonders einfach außerhalb der implantierbaren Anordnung ausgewertet werden. Dabei kann die Datenübermittlung an ein externes Gerät insbesondere im Rahmen von Einzeluntersuchungen eines Patienten durch einen Arzt sinnvoll sein. Demgegenüber ermöglicht eine Datenübermittlung an ein Datenzentrum eine kontinuierliche Überwachung des Zustands der implantierbaren Anordnung, die die Daten sendet, bzw. des Patienten, dem die Anordnung implantiert wurde. Auf diese Weise lassen sich im Rahmen der Telemedizin Ergebnisse gewinnen, die eine verbesserte Patientenbetreuung und/oder erhöhte Gerätesicherheit ermöglichen. Die Datenübermittlung an ein externes Gerät bzw. an ein Datenzentrum erfolgt insbesondere kabellos auf funkgestützten Weg. Zu diesem Zweck ist die implantierbare Anordnung in dieser Variante mit einem entsprechenden Funkmodul ausgestattet.

In einer Variante weist die implantierbare Anordnung eine mehrpolige Elektrode auf. Dabei stellt die erste Detektionseinrichtung einen ersten Elektrodenpol der mehrpoligen Elektrode dar, während die zweite Detektionseinrichtung einen zweiten Elektrodenpol der mehrpoligen Elektrode darstellt. Die mehrpolige Elektrode kann beispielsweise 2, 3, 4, 5 oder 6 Elektrodenpole aufweisen, wobei eine größere Anzahl an Elektrodenpolen grundsätzlich ebenfalls denkbar ist. Auf diese Weise lässt sich die implantierbare Anordnung beispielsweise mit einer flottierenden Elektrode ausgestalten, die über ihre unterschiedlichen Elektrodenpole sowohl atriale Herzsignale als auch ventrikuläre Herzsignale erfasst.

In einer Variante ist es angedacht, dass die mehrpolige Elektrode nicht nur die erste Detektionseinrichtung und die zweite Detektionseinrichtung umfasst, sondern zusätzlich auch die dritte Detektionseinrichtung.

In einer weiteren Variante ist die dritte Detektionseinrichtung selbst als mehrpolige Elektrode ausgestaltet. Das heißt, sie weist in dieser Variante eine Vielzahl (zwei oder mehr) von Elektrodenpolen auf. Jeweils einer dieser Elektrodenpole dient zur Detektion eines intrinsischen linksventrikulären Signals. Mithin können in dieser Variante unterschiedliche linksventrikuläre Signale und somit in Bezug auf ein jeweils unmittelbar zuvor erfasstes rechtsventrikuläres Signal (leicht) unterschiedliche interventrikuläre Überleitungszeiten ermittelt werden. In einer Variante ist es möglich, eine einheitliche interventrikuläre Überleitungszeit aus den zuvor ermittelten einzelnen interventrikulären Überleitungszeiten zu berechnen, beispielsweise durch eine Mittelwertbildung. Es ist auch denkbar, einzelne zuvor bestimmte individuelle interventrikuläre Überleitungszeiten stärker als andere der zuvor bestimmten individuellen Überleitungszeiten zu gewichten, wenn dies aufgrund der Anordnung der einzelnen Elektrodenpole innerhalb des Herzens eines Patienten physiologisch sinnvoll erscheint.

Die dritte Detektionseinrichtung kann beispielsweise als quadrupolare Elektrode ausgestaltet sein, also vier Elektrodenpole aufweisen, die jeweils ein linksventrikuläres Signal erfassen. Typischerweise werden die kardialen Signale, die für eine linksventrikuläre Aktivität indikativ sind, von einer Elektrode erfasst, die am Koronarsinus eines Herzens verankert ist, da eine unmittelbare Einführung der Elektrode in den linken Ventrikel aufgrund der dort herrschenden Druckverhältnisse regelmäßig nicht möglich ist. Ein Aspekt der vorliegenden Erfindung betrifft ein Verfahren zur Steuerung des Betriebs einer implantierbaren Anordnung zur Detektion elektrischer Signale eines menschlichen oder tierischen Herzens gemäß einem der Ansprüche 1-11.

Das Verfahren weist die nachfolgend erläuterten Schritte auf.

Zunächst wird in einer ersten Variante eine intrinsische rechtsatriale Aktivität mittels einer ersten Detektionsvorrichtung erfasst. Anschließend wird mittels einer zweiten Detektionseinrichtung eine unmittelbar auf die zuvor erfasste rechtsatriale Aktivität folgende intrinsische rechtsventrikuläre Aktivität erfasst. Nachfolgend wird die Zeit, die zwischen der intrinsischen rechtsatrialen Aktivität und der intrinsischen rechtsventrikulären Aktivität vergangen ist, ermittelt. Diese Zeit wird dann als atrioventrikuläre Überleitungszeit gespeichert.

Alternativ oder zusätzlich zu der vorstehend erläuterten ersten Variante kann die nachfolgend erläuterte zweite Variante des Verfahrens durchgeführt werden. Gemäß dieser Variante wird zunächst eine intrinsische rechtsventrikuläre Aktivität mittels einer zweiten Detektionseinrichtung erfasst. Ferner wird eine unmittelbar auf die zuvor erfasste rechtsventrikuläre Aktivität folgende intrinsische linksventrikuläre Aktivität mittels einer dritten Detektionseinrichtung erfasst. Nachfolgend wird die Zeit, die zwischen der intrinsischen rechtsventrikulären Aktivität und der intrinsischen linksventrikulären Aktivität vergangen ist, bestimmt. Diese Zeit wird dann als interventrikuläre Überleitungszeit gespeichert.

Ein Aspekt der vorliegenden Erfindung betrifft ein Computerprogrammprodukt mit einem computerlesbaren Code, der den Prozessor der implantierbaren Anordnung zur Detektion elektrischer Signale eines menschlichen oder tierischen Herzens gemäß einem der Ansprüche 1-11 dazu veranlasst, die nachfolgend erläuterten Verfahrensschritte auszuführen, wenn er auf dem Prozessor ausgeführt wird.

Zunächst wird in einer ersten Variante eine intrinsische rechtsatriale Aktivität mittels einer ersten Detektionsvorrichtung erfasst. Anschließend wird mittels einer zweiten Detektionseinrichtung eine unmittelbar auf die zuvor erfasste rechtsatriale Aktivität folgende intrinsische rechtsventrikuläre Aktivität erfasst. Nachfolgend wird die Zeit, die zwischen der intrinsischen rechtsatrialen Aktivität und der intrinsischen rechtsventrikulären Aktivität vergangen ist, ermittelt. Diese Zeit wird dann als atrioventrikuläre Überleitungszeit gespeichert.

Alternativ oder zusätzlich zu der vorstehend erläuterten ersten Variante kann die nachfolgend erläuterte zweite Variante des Verfahrens durchgeführt werden. Gemäß dieser Variante wird zunächst eine intrinsische rechtsventrikuläre Aktivität mittels einer zweiten Detektionseinrichtung erfasst. Ferner wird eine unmittelbar auf die zuvor erfasste rechtsventrikuläre Aktivität folgende intrinsische linksventrikuläre Aktivität mittels einer dritten Detektionseinrichtung erfasst. Nachfolgend wird die Zeit, die zwischen der intrinsischen rechtsventrikulären Aktivität und der intrinsischen linksventrikulären Aktivität vergangen ist, bestimmt. Diese Zeit wird dann als interventrikuläre Überleitungszeit gespeichert.

Die vorliegende Erfindung findet Einsatz in einem Verfahren zur Behandlung eines menschlichen oder tierischen Patienten, der eine solche Behandlung benötigt. Dieses Verfahren ist nicht Teil der vorliegenden Erfindung und wird mittels einer implantierbaren Anordnung zur Detektion elektrischer Signale des Herzens des Patienten, insbesondere einer implantierbaren Anordnung gemäß den obigen Erläuterungen, durchgeführt. Die implantierbare Anordnung weist einen Prozessor, eine Speichereinrichtung und drei Detektionseinrichtungen auf. Eine erste Detektionseinrichtung dient dabei zum Detektieren einer atrialen Aktivität. Eine zweite Detektionseinrichtung dient zum Detektieren einer rechtsventrikulären Aktivität. Eine dritte Detektionseinrichtung dient zum Detektieren einer linksventrikulären Aktivität. Das Verfahren weist die nachfolgend erläuterten Schritte auf.

Gemäß einer ersten Variante wird eine intrinsische rechtsatriale Aktivität mittels der ersten Detektionsvorrichtung erfasst. Anschließend wird eine auf die zuvor erfasste intrinsische rechtsatriale Aktivität unmittelbar folgende intrinsische rechtsventrikuläre Aktivität mittels der zweiten Detektionseinrichtung erfasst. Anschließend wird die Zeit, die zwischen der intrinsischen rechtsatrialen Aktivität und der intrinsischen rechtsventrikulären Aktivität vergangen ist, bestimmt. Diese Zeit wird dann als atrioventrikuläre Überleitungszeit gespeichert.

Alternativ oder zusätzlich zu der vorstehend erläuterten ersten Variante kann auch die nachfolgend erläuterte zweite Variante des Verfahrens durchgeführt werden. Gemäß der zweiten Variante wird zunächst eine intrinsische rechtsventrikuläre Aktivität mittels der zweite Detektionseinrichtung erfasst. Anschließend wird eine auf die zuvor erfasste rechtsventrikuläre Aktivität unmittelbar folgende intrinsische linksventrikuläre Aktivität mittels der dritten Detektionseinrichtung erfasst. Schließlich wird die Zeit, die zwischen der intrinsischen rechtsventrikulären Aktivität und der intrinsischen linksventrikulären Aktivität vergangen ist, bestimmt. Diese Zeit wird dann als interventrikuläre Überleitungszeit gespeichert.

Unabhängig davon, ob die erste und/oder zweite Variante des Verfahrens durchgeführt wird, werden die bestimmten Überleitungszeiten (also die atrioventrikuläre Überleitungszeit und/oder die interventrikuläre Überleitungszeit) nachfolgend dazu verwendet, die zeitliche Abfolge von Stimulationsimpulsen anzupassen, die von einer zur Abgabe derartiger Stimulationsimpulse geeigneten Anordnung abgegeben werden. Diese Abgabe kann dabei der Stimulation des Herzens des Patienten als Ersatz für ausgebliebene intrinsische kardiale Impulse bzw. Aktivitäten oder aber zur Unterstützung einer zu schwachen bzw. zu geringen intrinsischen kardialen Funktion dienen. Die Anordnung, die zur Abgabe von Stimulationsimpulsen geeignet ist, kann dabei dieselbe Anordnung sein, die auch zur Detektion der kardialen Signale eingesetzt wird. Das heißt, ein Gerät, wie beispielsweise ein Herzschrittmacher oder ein CRT-Gerät, kann sowohl zur Detektion kardialer Signale bzw. einer kardialen Aktivität als auch zur Stimulation desselben Herzens eingesetzt werden.

Sämtliche Varianten und alternativen Ausgestaltungen, die in Zusammenhang mit der implantierbaren Anordnung beschrieben wurden, sind beliebig miteinander kombinierbar und auf die beschriebenen Verfahren und das beschriebene Computerprogrammprodukt übertragbar. Ferner sind die beschriebenen Varianten der Verfahren beliebig miteinander kombinierbar und auf die jeweils anderen Verfahren sowie auf das Computerprogrammprodukt und die Anordnung übertragbar. In gleicher Weise sind die beschriebenen Varianten des Computerprogrammprodukts beliebig miteinander kombinierbar und auf die beschriebenen Verfahren und die beschriebene Anordnung übertragbar.

Weitere Details von Aspekten der Erfindung werden nachfolgend in Zusammenhang mit einem Ausführungsbeispiel und einer Zeichnung näher erläutert. Es zeigt:

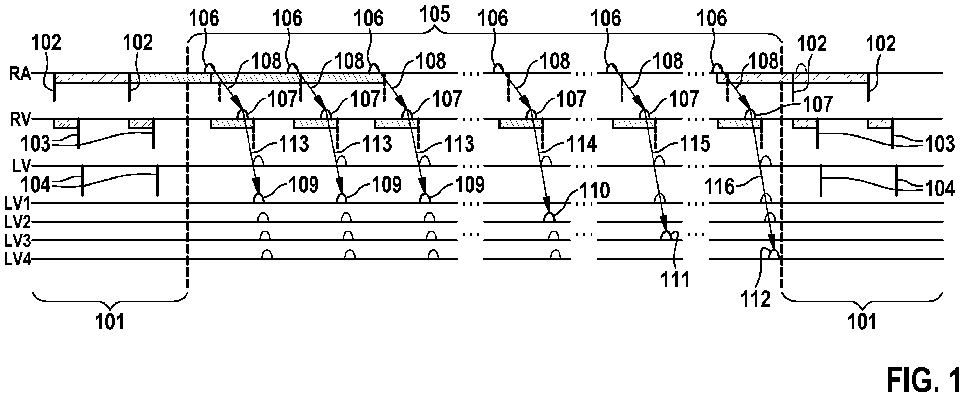

- Figur 1: einen schematischen Ablaufplan eines Verfahrens zur Bestimmung der atrioventrikulären Überleitungszeit und der interventrikulären Überleitungszeit mittels eines Herzschrittmachers.

Die Figur 1 zeigt einen schematischen Ablaufplan eines Programms zur Bestimmung der atrioventrikulären Überleitungszeit und der interventrikulären Überleitungszeit mittels eines Herzschrittmachers. Dabei geben die einzelnen Linien die Herzkammern an, in denen mittels des Herzschrittmachers ein Signal erfasst oder abgegeben werden kann. RA bezeichnet das rechte Atrium, RV den rechten Ventrikel und LV den linken Ventrikel. Da der Herzschrittmacher im Ausführungsbeispiel der Figur 1 eine quadrupolare linksventrikuläre Elektrode aufweist, lassen sich die linksventrikulären Signale in ein erstes linksventrikuläres Signal LV1, ein zweites linksventrikuläre Signal LV2, ein drittes linksventrikuläres Signal LV3 und ein viertes linksventrikuläres Signal LV4 unterscheiden.

In einem ersten, standardmäßig durchgeführten Betriebsprogramm 101 können erste Stimulationspulse 102 in das rechte Atrium, zweite Stimulationspulse 103 in den rechten Ventrikel und dritte Stimulationspulse 104 in den linken Ventrikel abgegeben werden.

In einem Messprogramm 105 wird die tatsächliche oder mögliche Abgabe derartiger Stimulationspulse 102, 103, 104 vollständig inhibiert, was durch die gestrichelten senkrechten Linien im zeitlichen Bereich des Messprogramms 105 dargestellt ist. Folglich kann während des Messprogramms 105, lediglich eine intrinsische Aktivität des Herzens des Patienten, der den Herzschrittmacher trägt, ermittelt werden.

Zur Erfassung einer derartigen intrinsischen kardialen Aktivität erfolgt in regelmäßigen Abständen eine rechtsatriale Signalerfassung 106. Ebenso erfolgt in regelmäßigen Abständen eine rechtsventrikuläre Signalerfassung 107. Ein zeitlicher Versatz zwischen den erfassten rechtsatrialen Signalen 106 und den unmittelbar folgenden, erfassten rechtsventrikulären Signalen 107 spiegelt die atrioventrikuläre Überleitungszeit 108 wieder.

Mittels der unterschiedlichen linksventrikulären Kanäle können erste linksventrikuläre Signale 109, zweite linksventrikuläre Signale 110, dritte linksventrikuläre Signale 111 und vierte linksventrikuläre Signale 112 erfasst werden. Dabei wird eine Erfassung jeweils nur eines Typs linksventrikulärer Signale zum selben Zeitpunkt gestattet. Eine Erfassung der anderen linksventrikulären Signale wird hingegen inhibiert. Es können zu einem Zeitpunkt also nur entweder erste linksventrikuläre Signale 109 oder zweite linksventrikuläre Signale 110 oder dritte linksventrikuläre Signale 111 oder vierte linksventrikuläre Signale 112 erfasst werden.

Ein erster zeitlicher Versatz zwischen den detektierten rechtsventrikulären Signalen 107 und den erfassten unmittelbar folgenden ersten linksventrikulären Signalen 109 stellt eine erste interventrikuläre Überleitungszeit 113 dar.

Ein zweiter zeitlicher Versatz zwischen den erfassten rechtsventrikulären Signalen 107 und den erfassten unmittelbar folgenden zweiten linksventrikulären Signalen 110 stellt eine zweite interventrikuläre Überleitungszeit 114 dar.

Ein dritter zeitlicher Versatz zwischen den erfassten rechtsventrikulären Signalen 107 und den unmittelbar folgenden dritten linksventrikulären Signalen 111 stellt eine dritte interventrikuläre Überleitungszeit 115 dar.

Schließlich stellt ein vierter zeitlicher Versatz zwischen den erfassten rechtsventrikulären Signale 107 und den unmittelbar folgenden vierten linksventrikulären Signalen 112 eine vierte interventrikuläre Überleitungszeit 116 dar.

Die erste interventrikuläre Überleitungszeit 113, die zweite interventrikuläre Überleitungszeit 114, die dritte interventrikuläre Überleitungszeit 115 und die vierte interventrikuläre Überleitungszeit 116 können gleich oder aber verschieden voneinander sein, je nachdem wie die einzelnen Elektroden, die zur Erfassung der unterschiedlichen linksventrikulären Signale eingesetzt werden, innerhalb des Herzens angeordnet sind und je nachdem wie die physiologische Reizweiterleitung in dem betrachteten Herz erfolgt.

Das Messprogramm 105 kann derart durchgeführt werden, dass eine interventrikuläre Überleitungszeit anhand verschiedener (gegebenenfalls unmittelbar aufeinander folgender) erfasster kardialer Signalgruppen ermittelt wird, wie dies beispielhaft für die erste interventrikuläre Überleitungszeit 113 dargestellt ist. So wird die erste interventrikuläre Überleitungszeit 113 anhand dreier Signalgruppen ermittelt, die jeweils aus einem rechtsventrikulären Signal 107 und einem ersten linksventrikulären Signal 109 bestehen.

Gleichfalls ist es möglich, lediglich einzelne kardiale Signale auszuwerten, um eine Überleitungszeit zu ermitteln, wie dies beispielhaft für die zweite interventrikuläre Überleitungszeit 114, die dritte interventrikuläre Überleitungszeit 115 und die vierte interventrikuläre Überleitungszeit 116 dargestellt ist.

Das Messprogramm 105 wird in dem Ausführungsbeispiel der Figur 1 dann durchgeführt, wenn der entsprechende Herzschrittmacher routinemäßig die Messung eines Signals der intrinsischen Herzaktivität bzw. die Bestimmung und/oder Anpassung einer Detektionsschwelle der implantierbaren Anordnung durchführt. Eine derartige Ermittlung erfolgt typischerweise einmal pro Tag. Mithin erfolgt auch eine Ermittlung der atrioventrikulären Überleitungszeit 108 und der unterschiedlichen interventrikulären Überleitungszeiten 113, 114, 115, 116 einmal pro Tag. Wenn die Überleitungszeiten ermittelt und die routinemäßig die Messung eines Signals der intrinsischen Herzaktivität bzw. die Bestimmung und/oder Anpassung einer Detektionsschwelle der implantierbaren Anordnung durchgeführt wurden, geht der Herzschrittmacher wieder in sein Standard-Betriebsprogramm 101 über, in dem rechtsatriale Stimulationsimpulse 102 sowie rechtsventrikuläre Stimulationsimpulse 103 und linksventrikulären Stimulationsimpulse 104 abgegeben werden können. Eine Inhibition dieser Stimulationsimpulse ist also nur während des Messprogramms 105 erforderlich und vorgesehen, um zu diesem Zeitpunkt tatsächlich nur die intrinsischen Herzsignale, die die intrinsische Herzaktivität in den unterschiedlichen Kompartimenten des Herzens widerspiegeln, zu erfassen.

## Patentansprüche

1. Implantierbare Anordnung zur Detektion elektrischer Signale eines menschlichen oder tierischen Herzens, aufweisend einen Prozessor, eine Speichereinrichtung, eine erste Detektionseinrichtung zum Detektieren einer atrialen Aktivität, eine zweite Detektionseinrichtung zum Detektieren einer rechtsventrikulären Aktivität, eine dritte Detektionseinrichtung zum Detektieren einer linksventrikulären Aktivität,
wobei
die Speichereinrichtung ein computerlesbares Programm aufweist, das den Prozessor dazu veranlasst, die folgenden Schritte in regelmäßigen Abständen automatisch auszuführen, wenn es auf dem Prozessor ausgeführt wird:
a1) Erfassen einer intrinsischen rechtsatrialen Aktivität (106) mittels der ersten Detektionseinrichtung,
b1) Erfassen einer intrinsischen rechtsventrikulären Aktivität (107) mittels der zweiten Detektionseinrichtung,
c1) Bestimmen der Zeit, die zwischen der intrinsischen rechtsatrialen Aktivität und der intrinsischen rechtsventrikulären Aktivität vergangen ist, und Speichern dieser Zeit als atrioventrikuläre Überleitungszeit (108),
und/oder
a2) Erfassen einer intrinsischen rechtsventrikulären Aktivität (107) mittels der zweiten Detektionseinrichtung,
b2) Erfassen einer intrinsischen linksventrikulären Aktivität (109, 110, 111, 112) mittels der dritten Detektionseinrichtung,
c2) Bestimmen der Zeit, die zwischen der intrinsischen rechtsventrikulären Aktivität und der intrinsischen linksventrikulären Aktivität vergangen ist, und Speichern dieser Zeit als interventrikuläre Überleitungszeit (113, 114, 115, 116),
wobei das Programm den Prozessor dazu veranlasst, die genannten Schritte einmal in einem Zeitraum von 3 Stunden bis 48 Stunden durchzuführen und
**dadurch gekennzeichnet, dass**
das Programm den Prozessor dazu veranlasst, die genannten Schritte während einer Bestimmung und/oder Anpassung einer Detektionsschwelle der implantierbaren Anordnung durchzuführen.

2. Implantierbare Anordnung nach Anspruch 1, **dadurch gekennzeichnet, dass** zusätzlich zu den Schritten a2) und b2) der Schritt a1) vor dem Schritt c2) durchgeführt wird.

3. Implantierbare Anordnung nach einem der vorhergehenden Ansprüche, **dadurch gekennzeichnet, dass** die implantierbare Anordnung auch zur Stimulation desselben menschlichen oder tierischen Herzens vorgesehen und eingerichtet ist, dessen Aktivität die implantierbare Anordnung erfassen kann.

4. Implantierbare Anordnung nach einem der vorhergehenden Ansprüche, **dadurch gekennzeichnet, dass** das Programm den Prozessor dazu veranlasst, eine atriale Stimulation (102) und eine ventrikuläre Stimulation (103, 104) so lange zu inhibieren, bis die atrioventrikuläre Überleitungszeit (108) und/oder die interventrikuläre Überleitungszeit (113, 114, 115, 116) bestimmt ist.

5. Implantierbare Anordnung nach einem der vorhergehenden Ansprüche, **dadurch gekennzeichnet, dass** das Programm den Prozessor dazu veranlasst, die genannten Schritte während einer routinemäßig durchgeführten Messung eines Signals der intrinsischen Herzaktivität der implantierbaren Anordnung durchzuführen.

6. Implantierbare Anordnung nach Anspruch 5, wobei die Messung eines Signals der intrinsischen Herzaktivität die Messung von mindestens einem Signalparameter aus Amplitude, Pulsbreite, Anzahl der Nulldurchgänge, Amplituden- und/oder Phasen-Frequenzspektrum und/oder Energie des Signals sowie die Parameter
- eines Modells des Signals (z.B. ARMA)
und/oder
- einer Transformation des Signal (z.B. Wavelet)
und/oder
Amplitude, Pulsbreite, Anzahl der Nulldurchgänge, Amplituden- und/oder Phasen-Frequenzspektrum und/oder Energie einer zeitlichen Ableitung des Signals umfasst.

7. Implantierbare Anordnung nach einem der vorhergehenden Ansprüche, **dadurch gekennzeichnet, dass** das Programm den Prozessor dazu veranlasst, eine Vielzahl von zuvor bestimmten atrioventrikulären Überleitungszeiten (108) und/oder interventrikulären Überleitungszeiten (113, 114, 115, 116) zu speichern.

8. Implantierbare Anordnung nach Anspruch 7, **dadurch gekennzeichnet, dass** das Programm den Prozessor dazu veranlasst, aus der Vielzahl gespeicherter atrioventrikulärer Überleitungszeiten (108) und/oder interventrikulärer Überleitungszeiten (113, 114, 115, 116) einen zeitlichen Verlauf der jeweiligen Überleitungszeit zu ermitteln.

9. Implantierbare Anordnung nach einem der vorhergehenden Ansprüche, **dadurch gekennzeichnet, dass** das Programm den Prozessor dazu veranlasst, die atrioventrikuläre Überleitungszeit (108) und/oder die interventrikuläre Überleitungszeit (113, 114, 115, 116) an ein externes Gerät oder an ein Datenzentrum zu übermitteln.

10. Implantierbare Anordnung nach einem der vorhergehenden Ansprüche, **dadurch gekennzeichnet, dass** die implantierbare Anordnung eine mehrpolige Elektrode aufweist, wobei die erste Detektionseinrichtung ein erster Elektrodenpol der mehrpoligen Elektrode ist und die zweite Detektionseinrichtung ein zweiter Elektrodenpol der mehrpoligen Elektrode ist.

11. Implantierbare Anordnung nach einem der vorhergehenden Ansprüche, **dadurch gekennzeichnet, dass** die dritte Detektionseinrichtung eine Vielzahl von Elektrodenpolen aufweist, die jeweils zur Detektion einer intrinsischen linksventrikulären Aktivität (109, 110, 111, 112) vorgesehen sind.

12. Verfahren zur Steuerung des Betriebs einer implantierbaren Anordnung zur Detektion elektrischer Signale eines menschlichen oder tierischen Herzens gemäß einem der vorherigen Ansprüche, mit den folgenden Schritten:
a1) Erfassen einer intrinsischen rechtsatrialen Aktivität (106) mittels einer ersten Detektionseinrichtung,
b1) Erfassen einer intrinsischen rechtsventrikulären Aktivität (107) mittels einer zweiten Detektionseinrichtung,
c1) Bestimmen der Zeit, die zwischen der intrinsischen rechtsatrialen Aktivität und der intrinsischen rechtsventrikulären Aktivität vergangen ist, und Speichern dieser Zeit als atrioventrikuläre Überleitungszeit (108),
und/oder
a2) Erfassen einer intrinsischen rechtsventrikulären Aktivität (107) mittels einer zweiten Detektionseinrichtung,
b2) Erfassen einer intrinsischen linksventrikulären Aktivität (109, 110, 111, 112) mittels einer dritten Detektionseinrichtung,
c2) Bestimmen der Zeit, die zwischen der intrinsischen rechtsventrikulären Aktivität und der intrinsischen linksventrikulären Aktivität vergangen ist, und Speichern dieser Zeit als interventrikuläre Überleitungszeit (113, 114, 115, 116),
wobei die genannten Schritte einmal in einem Zeitraum von 3 Stunden bis 48 Stunden durchgeführt werden und die genannten Schritte während einer Bestimmung und/oder Anpassung einer Detektionsschwelle der implantierbaren Anordnung durchgeführt werden.

13. Computerprogrammprodukt mit einem computerlesbaren Code, der den Prozessor der implantierbaren Anordnung zur Detektion elektrischer Signale eines menschlichen oder tierischen Herzens gemäß einem der Ansprüche 1-11 dazu veranlasst, die folgenden Schritte auszuführen, wenn er auf dem Prozessor ausgeführt wird:
a1) Erfassen einer intrinsischen rechtsatrialen Aktivität (106) mittels einer ersten Detektionseinrichtung,
b1) Erfassen einer intrinsischen rechtsventrikulären Aktivität (107) mittels einer zweiten Detektionseinrichtung,
c1) Bestimmen der Zeit, die zwischen der intrinsischen rechtsatrialen Aktivität und der intrinsischen rechtsventrikulären Aktivität vergangen ist, und Speichern dieser Zeit als atrioventrikuläre Überleitungszeit (108),
und/oder
a2) Erfassen einer intrinsischen rechtsventrikulären Aktivität (107) mittels einer zweiten Detektionseinrichtung,
b2) Erfassen einer intrinsischen linksventrikulären Aktivität (109, 110, 111, 112) mittels einer dritten Detektionseinrichtung,
c2) Bestimmen der Zeit, die zwischen der intrinsischen rechtsventrikulären Aktivität und der intrinsischen linksventrikulären Aktivität vergangen ist, und Speichern dieser Zeit als interventrikuläre Überleitungszeit (113, 114, 115, 116),
wobei die genannten Schritte einmal in einem Zeitraum von 3 Stunden bis 48 Stunden durchgeführt werden und die genannten Schritte während einer Bestimmung und/oder Anpassung einer Detektionsschwelle der implantierbaren Anordnung durchgeführt werden.

## Claims

1. An implantable system for detecting electrical signals of a human heart or an animal heart, the implantable system comprising:
a processor, a memory unit, a first detection unit for detecting an atrial activity, a second detection unit for detecting a right ventricular activity, and a third detection unit for detecting a left ventricular activity,
said memory unit including a computer-readable program prompting said processor to automatically carry out steps at regular intervals upon said program being executed on said processor, said steps including:
a1) detecting an intrinsic right atrial activity (106) by using said first detection unit;
b1) detecting an intrinsic right ventricular activity (107) by using said second detection unit; and
c1) determining a time having passed between the intrinsic right atrial activity and the intrinsic right ventricular activity, and storing said time as an atrioventricular conduction time (108);
and/or
a2) detecting an intrinsic right ventricular activity (107) by using said second detection unit;
b2) detecting an intrinsic left ventricular activity (109, 110, 111, 112) by using said third detection unit; and
c2) determining a time having passed between the intrinsic right ventricular activity and the intrinsic left ventricular activity, and storing said time as an interventricular conduction time (113, 114, 115, 116);
said program being configured to prompt said processor to carry out said steps once within a time period of from 3 hours to 48 hours and
**characterized in that**
said program being configured to prompt said processor to carry out said steps during a determination and/or an adaptation of a detection threshold of the implantable system.

2. The implantable system according to claim 1, **characterized in that** step a1), in addition to steps a2) and b2), is carried out prior to step c2).

3. The implantable system according to one of the preceding claims, **characterized in that** the implantable system is provided and configured to stimulate the human heart or animal heart having the activity to be detected by the implantable system.

4. The implantable system according to one of the preceding claims, **characterized in that** said program prompts said processor to inhibit an atrial stimulation (102) and a ventricular stimulation (103, 104) until a determination of at least one of said atrioventricular conduction time (108) or said interventricular conduction time (113, 114, 115, 116).

5. The implantable system according to one of the preceding claims, **characterized in that** said program prompts said processor to carry out said steps during a routine measurement of a signal of the intrinsic cardiac activity of the implantable system.

6. The implantable system according to claim 5, wherein said measurement of a signal of the intrinsic cardiac activity encompasses a measurement of at least one signal parameter of amplitude, pulse width, number of zero crossings, at least one of amplitude or phase frequency spectrum or energy of the signal, as well as parameters of at least one of:
- a model of the signal (such as ARMA), or
- a transformation of the signal (such as wavelet), or
an amplitude, pulse width, number of zero crossings, at least one of amplitude or phase frequency spectrum or energy of a time derivative of the signal.

7. The implantable system according to one of the preceding claims, **characterized in that** said program prompts said processor to store a plurality of at least one of previously determined atrioventricular conduction times (108) or interventricular conduction times (113, 114, 115, 116).

8. The implantable system according to claim 7, **characterized in that** said program prompts said processor to ascertain a chronological progression of a particular conduction time from said plurality of at least one of stored atrioventricular conduction times (108) or interventricular conduction times (113, 114, 115, 116).

9. The implantable system according to one of the preceding claims, **characterized in that** said program prompts said processor to transmit at least one of an atrioventricular conduction time (108) or an interventricular conduction time (113, 114, 115, 116) to an external device or to a data center.

10. The implantable system according to one of the preceding claims, **characterized by** further comprising a multipolar electrode, said first detection unit being a first electrode pole of said multipolar electrode, and said second detection unit being a second electrode pole of said multipolar electrode.

11. The implantable system according to one of the preceding claims, **characterized in that** said third detection unit includes a plurality of electrode poles each provided for detecting an intrinsic left ventricular activity (109, 110, 111, 112).

12. A method for controlling an operation of an implantable system for detecting electrical signals of a human heart or an animal heart, the method comprising the following steps:
a1) detecting an intrinsic right atrial activity (106) by using a first detection unit;
b1) detecting an intrinsic right ventricular activity (107) by using a second detection unit; and
c1) determining a time having passed between the intrinsic right atrial activity and the intrinsic right ventricular activity, and storing the time as an atrioventricular conduction time (108);
and/or
a2) detecting an intrinsic right ventricular activity (107) by using the second detection unit;
b2) detecting an intrinsic left ventricular activity (109, 110, 111, 112) by using a third detection unit; and
c2) determining a time having passed between the intrinsic right ventricular activity and the intrinsic left ventricular activity, and storing the time as an interventricular conduction time (113, 114, 115, 116);
wherein said steps are carried out once within a time period of from 3 hours to 48 hours and said steps are carried out during at least one of a determination or an adaptation of a detection threshold of the implantable system.

13. A non-transitory computer program product including computer-readable code prompting the processor of the implantable system for detecting electrical signals of a human heart or an animal heart according to one of claims 1-11 to carry out the following steps upon the code being executed on the processor:
a1) detecting an intrinsic right atrial activity (106) by using a first detection unit;
b1) detecting an intrinsic right ventricular activity (107) by using a second detection unit; and
c1) determining a time having passed between the intrinsic right atrial activity and the intrinsic right ventricular activity, and storing the time as an atrioventricular conduction time (108);
and/or
a2) detecting an intrinsic right ventricular activity (107) by using the second detection unit;
b2) detecting an intrinsic left ventricular activity (109, 110, 111, 112) by using a third detection unit; and
c2) determining a time having passed between the intrinsic right ventricular activity and the intrinsic left ventricular activity, and storing the time as an interventricular conduction time (113, 114, 115, 116);
wherein said steps are carried out once within a time period of from 3 hours to 48 hours and said steps are carried out during at least one of a determination or an adaptation of a detection threshold of the implantable system.

## Revendications

1. Ensemble implantable permettant la détection de signaux électriques d'un coeur humain ou animal, présentant un processeur, un dispositif de mémoire, un premier dispositif de détection permettant la détection d'une activité atriale, un deuxième dispositif de détection permettant la détection d'une activité ventriculaire droite, un troisième dispositif de détection permettant la détection d'une activité ventriculaire gauche, dans lequel
le dispositif de mémoire présente un programme lisible par ordinateur qui, lorsqu'il est exécuté sur le processeur, fait en sorte que le processeur exécute automatiquement les étapes suivantes à intervalles réguliers :
a1) détection d'une activité atriale droite intrinsèque (106) au moyen du premier dispositif de détection,
b1) détection d'une activité ventriculaire droite intrinsèque (107) au moyen du deuxième dispositif de détection,
c1) détermination du temps qui s'est écoulé entre l'activité atriale droite intrinsèque et l'activité ventriculaire droite intrinsèque et enregistrement de ce temps en tant que temps de conduction auriculo-ventriculaire (108),
et/ou
a2) détection d'une activité ventriculaire droite intrinsèque (107) au moyen du deuxième dispositif de détection,
b2) détection d'une activité ventriculaire gauche intrinsèque (109, 110, 111, 112) au moyen du troisième dispositif de détection,
c2) détermination du temps qui s'est écoulé entre l'activité ventriculaire droite intrinsèque et l'activité ventriculaire gauche intrinsèque et enregistrement de ce temps en tant que temps de conduction inter ventriculaire (113, 114, 115, 116),
dans lequel le programme fait en sorte que le processeur exécute lesdites étapes une fois pendant un intervalle de temps de 3 heures à 48 heures, et
**caractérisé en ce que**
le programme fait en sorte que le processeur exécute lesdites étapes pendant une détermination et/ou une adaptation d'un seuil de détection de l'ensemble implantable.

2. Ensemble implantable selon la revendication 1, **caractérisé en ce que** l'étape a1) est exécutée en complément des étapes a2) et b2) avant l'étape c2).

3. Ensemble implantable selon l'une des revendications précédentes, **caractérisé en ce que** l'ensemble implantable est prévu et conçu également pour la stimulation du même coeur humain ou animal, dont l'activité peut être détectée par l'ensemble implantable.

4. Ensemble implantable selon l'une des revendications précédentes, **caractérisé en ce que** le programme fait en sorte que le processeur inhibe une stimulation atriale (102) et une stimulation ventriculaire (103, 104) jusqu'à ce que le temps de conduction auriculo-ventriculaire (108), et/ou le temps de conduction inter ventriculaire (113, 114, 115, 116), soit déterminé.

5. Ensemble implantable selon l'une des revendications précédentes, **caractérisé en ce que** le programme fait en sorte que le processeur exécute lesdites étapes pendant une mesure d'un signal de l'activité cardiaque intrinsèque de l'ensemble implantable exécutée de manière routinière.

6. Ensemble implantable selon la revendication 5, dans lequel la mesure d'un signal de l'activité cardiaque intrinsèque comprend la mesure d'au moins un paramètre signalétique parmi une amplitude, une largeur d'impulsion, un nombre de passages par zéro, un spectre d'amplitudes et/ou de fréquences de phases, et/ou une énergie du signal, ainsi que les paramètres
- un modèle du signal (par exemple, ARMA)
et/ou
- une transformation du signal (par exemple, ondelette)
et/ou
une amplitude, une largeur d'impulsion, un nombre de passages par zéro, un spectre d'amplitudes et/ou de fréquences de phases, et/ou une énergie d'une déviation temporelle du signal.

7. Ensemble implantable selon l'une des revendications précédentes, **caractérisé en ce que** le programme fait en sorte que le processeur enregistre une multiplicité de temps de conduction auriculo-ventriculaires (108) et/ou de temps de conduction inter ventriculaires (113, 114, 115, 116) déterminés auparavant.

8. Ensemble implantable selon la revendication 7, **caractérisé en ce que** le programme fait en sorte que le processeur détermine, à partir de la multiplicité des temps de conduction auriculo-ventriculaires (108) et/ou des temps de conduction inter ventriculaires (113, 114, 115, 116) enregistrés, une évolution dans le temps du temps de conduction respectif.

9. Ensemble implantable selon l'une des revendications précédentes, **caractérisé en ce que** le programme fait en sorte que le processeur transmette le temps de conduction auriculo-ventriculaire (108), et/ou le temps de conduction inter ventriculaire (113, 114, 115, 116) à un appareil externe ou à centre de données.

10. Ensemble implantable selon l'une des revendications précédentes, **caractérisé en ce que** l'ensemble implantable présente une électrode multipolaire, où le premier dispositif de détection est un premier pôle d'électrode de l'électrode multipolaire et le deuxième dispositif de détection est un deuxième pôle d'électrode de l'électrode multipolaire.

11. Ensemble implantable selon l'une des revendications précédentes, **caractérisé en ce que** le troisième dispositif de détection présente une multiplicité de pôles d'électrode qui sont respectivement prévus pour la détection d'une activité ventriculaire gauche intrinsèque (109, 110, 111, 112).

12. Procédé de commande du fonctionnement d'un ensemble implantable permettant la détection de signaux électriques d'un coeur humain ou animal selon l'une des revendications précédentes, avec les étapes suivantes :
a1) détection d'une activité atriale droite intrinsèque (106) au moyen d'un premier dispositif de détection,
b1) détection d'une activité ventriculaire droite intrinsèque (107) au moyen d'un deuxième dispositif de détection,
c1) détermination du temps qui s'est écoulé entre l'activité atriale droite intrinsèque et l'activité ventriculaire droite intrinsèque et enregistrement de ce temps en tant que temps de conduction auriculo-ventriculaire (108), et/ou
a2) détection d'une activité ventriculaire droite intrinsèque (107) au moyen d'un deuxième dispositif de détection,
b2) détection d'une activité ventriculaire gauche intrinsèque (109, 110, 111, 112) au moyen d'un troisième dispositif de détection,
c2) détermination du temps qui s'est écoulé entre l'activité ventriculaire droite intrinsèque et l'activité ventriculaire gauche intrinsèque et enregistrement de ce temps en tant que temps de conduction inter ventriculaire (113, 114, 115, 116),
dans lequel lesdites étapes sont exécutées une fois dans un intervalle de temps de 3 heures à 48 heures et lesdites étapes sont exécutées pendant une détermination et/ou une adaptation d'un seuil de détection de l'ensemble implantable.

13. Produit-programme informatique avec un code lisible par ordinateur, qui, lorsqu'il est exécuté sur le processeur, fait en sorte que le processeur de l'ensemble implantable pour la détection de signaux électriques d'un coeur humain ou animal selon les revendications 1 à 11 exécute les étapes suivantes :
a1) détection d'une activité atriale droite intrinsèque (106) au moyen d'un premier dispositif de détection,
b1) détection d'une activité ventriculaire droite intrinsèque (107) au moyen d'un deuxième dispositif de détection,
c1) détermination du temps qui s'est écoulé entre l'activité atriale droite intrinsèque et l'activité ventriculaire droite intrinsèque et enregistrement de ce temps en tant que temps de conduction auriculo-ventriculaire (108),
et/ou
a2) détection d'une activité ventriculaire droite intrinsèque (107) au moyen d'un deuxième dispositif de détection,
b2) détection d'une activité ventriculaire gauche intrinsèque (109, 110, 111, 112) au moyen d'un troisième dispositif de détection,
c2) détermination du temps qui s'est écoulé entre l'activité ventriculaire droite intrinsèque et l'activité ventriculaire gauche intrinsèque et enregistrement de ce temps en tant que temps de conduction inter ventriculaire (113, 114, 115, 116),
dans lequel lesdites étapes sont exécutées une fois dans un intervalle de temps de 3 heures à 48 heurs et lesdites étapes sont exécutées pendant une détermination et/ou une adaptation d'un seuil de détection de l'ensemble implantable.
